# EUROPEAN PATENT APPLICATION

(11) **EP 3 682 849 A1**
(43) Date of publication of application: **22.07.2020**
(21) Application number: 20151590.5
(22) Date of filing: 14.01.2020
(51) Int. Cl.: A61F 2/12

(54) **BREAST IMPLANT WITH MULTIPLE SALINE AND/OR SILICONE FILLABLE COMPARTMENTS**

(30) Priority: 15.01.2019 US 201916248412
(71) Applicant: BIOSENSE WEBSTER (ISRAEL) LTD., 2066717 Yokneam (IL)
(72) Inventor: GOVARI, Assaf, 2066717 Yokneam (IL); ALGAWI, Yehuda, 2066717 Yokneam (IL)
(74) Representative: Small, Gary James

(57) **Abstract**

A breast implant (21a) includes a flexible shell (22a) and multiple compartments (23a). The flexible shell is configured for implantation within a breast of a human subject. The multiple compartments, which are located inside the shell, are configured to be filled with respective filling materials including at least two of (i) a saline solution, (ii) a silicone foam and (iii) a selected mixture of the saline solution and the silicone foam. At least one of the compartments has a valve (25) that is configured to allow changing an amount of filled saline solution in the compartment.

## Description

### FIELD OF THE INVENTION

The present invention relates generally to medical implants, and particularly to breast implants.

### BACKGROUND OF THE INVENTION

A breast implant can be either inserted in a human breast or attached on the breast, e.g., in order to replace tissue that has been medically removed in an operation such as a mastectomy, or for cosmetic purposes. The purpose of the breast implant is to restore to the breast its external form, including its tactile feel and weight.

Various technologies are employed to form breast implants. For example, U.S. Patent Application Publication 2003/0074084 describes a method of breasts reconstruction that utilizes a breast prosthesis having a plurality of chambers or compartments distributed through a body member or shell in the form of a breast. The chambers are disposed along the superior, lateral and inferior surfaces, as well as in the interior, of the body member. The chambers are differentially pressurized or filled in order to control the shape of the prosthesis upon implantation thereof. Valves are provided for regulating the flow of fluid into and from the chambers. The prosthesis and the fill levels of the respective chambers may be selected by computer.

As another example, U.S. Patent Application Publication 2015/0351900 describes human implantable tissue expanders that comprise an inner foam filling enclosed within a substantially non-stretchable resilient expansion restricting layer configured to retain a shape and/or volume of said foam filling upon changes of ambient pressure and/or temperature, and an outer shell comprising one or more layers formed of a resilient material.

U.S. Patent Application Publication 2011/0264213 describes an implant for augmenting or reconstructing breasts that has independent expandable compartments filled during manufacture or during or after surgery, with an external silicone membrane internally and/or externally coated with a foam covered with microcrystals or nanocrystals incorporated into the same membrane, with an inner space divided into compartments that are or can be filled with gel, liquid, air or gas. A chamber for the projection of the body of the breast is having a conical structure within the main chamber. The inner and outer chambers are independently filled to allow adjustments of the implant through filling valves, in order to project the region of the areola and nipple, the body or other region of the breast for correcting ptosis.

### SUMMARY OF THE INVENTION

An embodiment of the present invention that is described hereinafter provides a breast implant including a flexible shell and multiple compartments. The flexible shell is configured for implantation within a breast of a human subject. The multiple compartments, which are located inside the shell, are configured to be filled with respective filling materials including at least two of (i) a saline solution, (ii) a silicone foam and (iii) a selected mixture of the saline solution and the silicone foam. At least one of the compartments has a valve that is configured to allow changing an amount of filled saline solution in the compartment.

In some embodiments, the saline solution and the silicone foam have substantially the same refractive index up to a given tolerance.

In some embodiments, the valve is configured to allow changing the amount of filled saline solution in the compartment after implantation.

There is additionally provided, in accordance with an embodiment of the present invention, a method for manufacturing a breast implant, the method including producing a flexible shell, configured for implantation within a breast of a human subject and including multiple compartments. The compartments are filled with respective filling materials including at least two of (i) a saline solution, (ii) a silicone foam, and (iii) a selected mixture of the saline solution and the silicone foam. In at least one of the compartments a valve is fitted that is configured to allow changing an amount of filled saline solution in the compartment.

There is further provided, in accordance with an embodiment of the present invention, a method including implanting, in a breast of a human subject, a breast implant including a flexible shell and multiple compartments that are located inside the shell and are configured to be filled with respective filling materials including at least two of (i) a saline solution, (ii) a silicone foam and (iii) a selected mixture of the saline solution and the silicone foam. At least one of the compartments has a valve that is configured to allow changing an amount of filled saline solution in the compartment. At least one breast implant property, selected from a group of breast implant properties consisting of the size and shape of the breast implant, is changed by changing the amount of filled saline solution in the compartment after implantation.

The present invention will be more fully understood from the following detailed description of the embodiments thereof, taken together with the drawings in which:

### BRIEF DESCRIPTION OF THE DRAWINGS

Figs. 1a and 1b are schematic sectional illustrations of a human female breasts, each with a multi-compartment breast implant, in accordance with embodiments of the invention; and
Fig. 2 is a flow chart that schematically illustrates a method for manufacturing the breast implant of Fig. 1, in accordance with an embodiment of the present invention.

### DETAILED DESCRIPTION OF EMBODIMENTS

### OVERVIEW

Commonly used breast implants are available at a given size and shape. At present, it is very difficult to change the size and/or shape of a silicone breast implant once the implant has been installed.

Embodiments of the present invention provide an implantable device that is used as a breast implant with an adaptable size and shape. The device comprises a sealed flexible shell that is configured for implantation within a breast of a human subject. The shell further comprises multiple sealed flexible compartments, one or more of which of which are fillable after implantation through a valve in the compartment's shell.

A compartment of the disclosed breast implant may be filled with silicone foam, saline solution, or a selected mixture of a silicone foam and saline solution. In an embodiment, the compartment is first partially filled with silicone foam and saline solution is injected afterwards.

Because the silicone foam and the saline have substantially the same refractive index, it is not apparent in transparent shells that the implant actually has two separate materials.

Embodiments of the present invention enable the addition or extraction of saline solution from different compartments, giving a physician the ability to control the shape and behavior (e.g., response to physical conditions) of the implant.

As the silicone foam and the saline are both under relatively high pressure (∼5 atmospheres), in some embodiments, saline is addied to a compartment using a pump that can flow the typically small amount of required saline.

The disclosed technique for manufacturing and filling a multi-compartment breast implant may enhance the capabilities of a physician to match an implant to a female subject and therefore ease the adaptation of the female subject to such an implant.

### IMPLANT DESCRIPTION

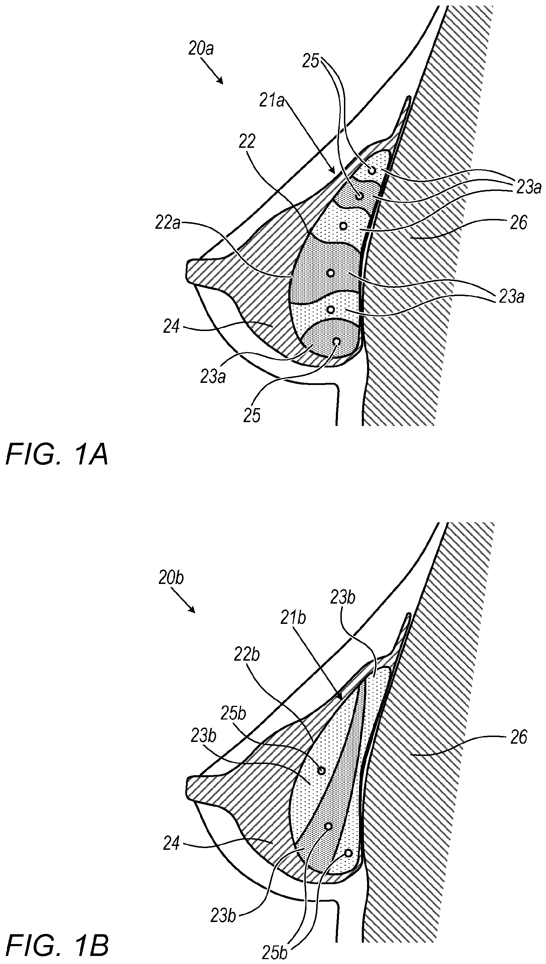

Figs. 1a and 1b are schematic sectional illustrations of a human female breasts 20a and 20b with a multi-compartment breast implants 21a and 21b, respectively, in accordance with embodiments of the invention, in accordance with an embodiment of the present invention.

As Fig. 1A shows, implant 21a comprises a shell 22a divided into compartments 23a, whereas compartments 23a are filled with either silicone foam or saline solution, or a mixture of the two.

In the disclosed embodiment, breast implant 21a is positioned as a subglandular implant between breast tissue 24 and a pectoralis major muscle 26. In alternative embodiments, breast implant 21a may be positioned either as a subfascial, subpectoral, or submuscular implant, referring to different positions of the implant relative to pectoralis major muscle 26, as will be understood by those skilled in the art.

In Fig. 1B, implant 21b comprises a shell 22b divided into compartments 23b. As seen, different embodiments, such as shown in Figs. 1A and 1B, may have varying number, shape, arrangement and content of the compartments.

In the shown embodiments, each compartment 23a or 23b has a valve 25 through which saline solution may be added to, or removed from the compartment, in order to optimize, for example, the shape of implants 21a and 21b.

In some embodiments, the silicone foam and the saline have substantially the same refractive index up to a given tolerance, so that it is not apparent in transparent shells that the implant actually has two separate materials. For example, the difference in refractive indices is up to 10% at yellow light wavelength of 580 nm, i.e., with silicone foam having a refractive index in the range of [1.2-1.5].

The example shown in Fig. 1 is chosen purely for the sake of conceptual clarity. Embodiments of the present invention may apply to any implant design that contains multiple-sealed flexible compartments.

### MANUFACTURING BREAST IMPLANTS WITH MULTIPLE SALINE AND/OR SILICONE FILLABLE COMPARTMENTS

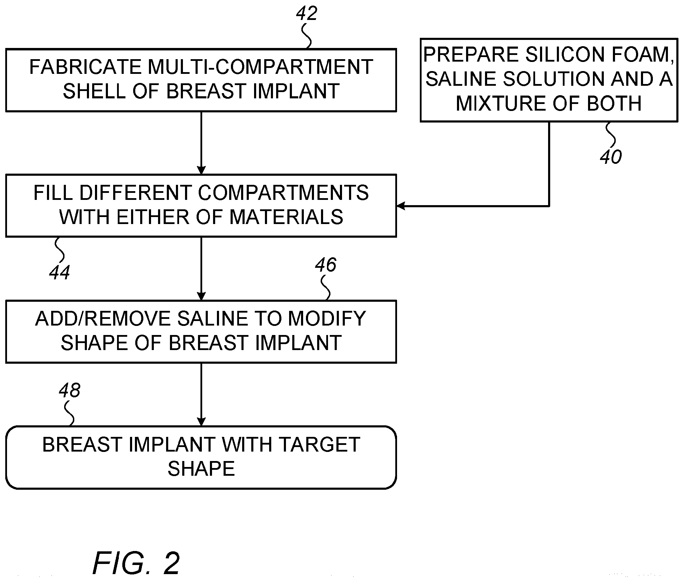

Fig. 2 is a flow chart that schematically illustrates a method for manufacturing the breast implant of Fig. 1, in accordance with an embodiment of the present invention. The method includes two manufacturing processes, wherein during one process the filling materials are prepared at a material preparation step 40. As noted above, the required materials are silicone foam, saline solution, and a mixture of silicone foam and saline solution. In an embodiment, a silicone foam is created inside a shell, in a chemical process that requires increased temperature (e.g., of 160° Celsius). Saline is added into the shell, before and/or after implantation in a body of a patient. In an embodiment, the saline is added by pumping the saline via a valve in the shell.

At a shell fabricating step 42, a multi-compartment (i.e., multi-pouch) shell is produced by means described in the literature. Next, the compartments of the shell are filled with any of the materials prepared in step 40, at a multi-compartment shell filling step 44. During the next step in the embodiment (shown as part of the manufacturing process, but which can also be performed during a medical procedure) saline solution is added or removed from at least one compartment 23, so as to modify the shape of implant 21a and/or 21b, at a shape modification step 46. The result 48 of the manufacturing process described above is an implant having a target shape.

The example flow chart shown in Fig. 2 is chosen purely for the sake of conceptual clarity. The filling materials, and the number and shape of compartments 23a and/or 23b (i.e., pouches) may vary, so as, for example, to modify the weight of the implant as well. Other manufacturing steps may be included, such as filtration and temperature settings, which for clarity are not shown.

Although the embodiments described herein mainly address breast implants, the methods and systems described herein can also be used in other applications, in which a shape of an implant needs to be controlled post implantation.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A breast implant, comprising:
a flexible shell, configured for implantation within a breast of a human subject; and
multiple compartments that are located inside the shell and are configured to be filled with respective filling materials comprising at least two of (i) a saline solution, (ii) a silicone foam and (iii) a selected mixture of the saline solution and the silicone foam, wherein at least one of the compartments has a valve that is configured to allow changing an amount of filled saline solution in the compartment.

2. The breast implant according to claim 1, wherein the saline solution and the silicone foam have substantially the same refractive index up to a given tolerance.

3. The breast implant according to claim 1, wherein the valve is configured to allow changing the amount of filled saline solution in the compartment after implantation.

4. A method for manufacturing a breast implant, the method comprising:
producing a flexible shell, configured for implantation within a breast of a human subject and comprising multiple compartments;
filling the compartments with respective filling materials comprising at least two of (i) a saline solution, (ii) a silicone foam, and (iii) a selected mixture of the saline solution and the silicone foam; and
fitting in at least one of the compartments a valve that is configured to allow changing an amount of filled saline solution in the compartment.

5. The method according to claim 4, wherein filling the compartments comprises filling the compartments with saline solution and silicone foam that have substantially the same refractive index up to a given tolerance.

6. A method, comprising:
implanting, in a breast of a human subject, a breast implant comprising:
a flexible shell; and
multiple compartments that are located inside the shell and are configured to be filled with respective filling materials comprising at least two of (i) a saline solution, (ii) a silicone foam and (iii) a selected mixture of the saline solution and the silicone foam, wherein at least one of the compartments has a valve that is configured to allow changing an amount of filled saline solution in the compartment; and
changing at least one breast implant property, selected from a group of breast implant properties consisting of the size and shape of the breast implant, by changing the amount of filled saline solution in the compartment after implantation.
